(19) Europäisches Patentamt
European Patent Office
Office européen des brevets

(11) Veröffentlichungsnummer: **0 295 405 A2**

(12) # EUROPÄISCHE PATENTANMELDUNG

(21) Anmeldenummer: **88106988.4**

(22) Anmeldetag: **01.05.88**

(51) Int. Cl.⁴: **A61L 2/18 , A01N 47/28 , C11D 3/48**

(30) Priorität: **17.06.87 DE 3720228**

(43) Veröffentlichungstag der Anmeldung: **21.12.88 Patentblatt 88/51**

(84) Benannte Vertragsstaaten: **AT BE CH DE FR GB IT LI NL**

(71) Anmelder: **CHEMISCHE FABRIK KREUSSLER & CO. GMBH**
**Rheingaustrasse 87-93**
**D-6200 Wiesbaden 12(DE)**

(72) Erfinder: **Hasenclever, Kaspar D.**

**D-6204 Taunusstein(DE)**

(74) Vertreter: **Weber, Dieter, Dr. et al**
**Willrath Weber und Seiffert Postfach 6145**
**Gustav-Freytag-Strasse 25**
**D-6200 Wiesbaden 1(DE)**

(54) **Verfahren und Mittel zur Desinfektion von Chemischreinigungsmaschinen.**

(57) Zur Vermeidung eines muffigen Geruches bei in Chemischreinigungsmaschinen gereinigten Textilien wird dem in den Chemischreinigungsmaschinen verwendeten Lösemittel zusammen mit einem Tensid ein durch 1 bis 4 Hydroxymethylgruppen substituierter Harnstoff zugesetzt. Ein Mittel zur Durchführung des Verfahrens besteht aus wenigstens einem solchen Harnstoffderivat, gegebenenfalls in einem Lösemittel oder im Gemisch mit einem als Reinigungsverstärker verwendbaren Tensid.

EP 0 295 405 A2

Xerox Copy Centre

## Verfahren und Mittel zur Desinfektion von Chemischreinigungsmaschinen

Die Erfindung betrifft ein Verfahren und Mittel zur Desinfektion von Chemischreinigungsmaschinen.

Chemischreinigungsmaschinen werden normalerweise mit einem Lösemittel Tetrachlorethen (Perchlorethylen) oder Trichlortrifluorethan betrieben. Da die Lösemittel für die Bereiche Luft und Wasser umweltgefährdend sind, sind die Chemischreinigungsmaschinen so konstruiert, daß die verwendeten Lösemittel innerhalb der Maschinen durch Destillation regeneriert und aus den Textilien, die mit den Lösemitteln in Kontakt kamen, in einem geschlossenen System aus Verdunstung und Kondensation zurückgewonnen werden.

Um den dazu erforderlichen Energieaufwand zu mindern, wird bei neueren Chemischreinigungsmaschinen nicht mehr mit Dampf oder Strom geheizt, um das Lösemittel zu verdunsten, und nicht mehr mit Wasser gekühlt, um es zu kondensieren, sondern es wird das Wärmepumpenprinzip angewandt. Dies hat zur Folge, daß das Temperaturniveau während der "Trocknung" der gereinigten Textilien von früher etwa 90 bis 110 °C auf 45 bis 55 °C gesenkt wurde.

Zur weiteren Erhöhung der Umweltfreundlichkeit von Chemischreinigungsmaschinen wurden diese in jüngerer Zeit auch insofern konstruktiv verändert, daß zum Ende des Trocknungsprozesses die lösemittelhaltige Restluft nicht mehr durch Frischluft verdrängt wird, was im übrigen eine Behandlung der Abluft mit Aktivkohlefiltern erforderlich machte, sondern in einem geschlossenen System gehalten und durch Abkühlung auf etwa -15 °C lösemittelarm gemacht wird.

Beide Änderungen, das Wärmepumpenprinzip mit der verbundenen Senkung der Trocknungstemperatur und der geschlossene Luftkreislauf, führen zusammen dazu, daß der Luftkreislauf währen der Trocknung stärker keimbelastet ist und vor allem die Maschinenbauteile im Kondensationsbereich, wie die Wärmetauscher für das Kühlen der Luft, bakteriell kontaminiert sind. Die Folge hiervon ist, daß in solchen Chemischreinigungsmaschinen bereits nach einer Nutzungsdauer von etwa einer Woche darin gereinigte Textilien einen faulen, muffigen Geruch annehmen. Dieser kommt hauptsächlich dadurch zustande, daß die Wasserphase des bei Chemischreinigungsmaschinen notwendigen Wasserabscheiders, der nach der Destillation und während der Trocknung das zurückgewonnene Lösemittel vom ebenfalls kondensierten Wasser trennt, mit Mikroorganismen verseucht ist. Infolge der normalen Betriebstemperatur um 30 °C herum können diese sich gut vermehren.

Wenn den Chemischreinigungsmaschinen aus Umweltschutzgründen noch Abwasseraufbereitungsanlagen nachgeschaltet sind, werden auch diese mikrobiologisch kontaminiert, wobei sehr oft an den in solchen Anlagen verwendeten Adsorbentien Pilzüberwachsungen auftreten, die eine einwandfreie Adsorption des Restlösemitteln aus dem Abwasser verhindern.

Beispielsweise aus der DE-PS 3 111 158 ist die Verwendung desinfizierender Reinigungsverstärker in Chemischreinigungsmaschinen bekannt, um Krankheitserreger abzutöten und eine Pigmentvergrauung der zu reinigenden Textilien zu vermindern. Die Verwendung der beschriebenen desinfizierenden Reinigungsverstärker hat jedoch den Nachteil, daß die Desinfektionswirkung nur in Maschinenbereichen auftritt, die von dem flüssigen Lösemittel, in welchem der desinfizierende Reinigungsverstärker gelöst ist, berührt werden. In der Gasphase der Chemischreinigungsmaschine dagegen sind die nichtflüchtigen desinfizierenden Reinigungsverstärker gemäß der DE-PS 3 111 158 nicht wirksam. Daher kann mit solchen Mitteln zwar das Eintreten einer mikrobiologischen Kontamination vermindert werden, doch kann eine dennoch eingetretene mikrobiologische Kontamination, wie auf den Wärmetauschern für das Kühlen der Luft, nicht wieder abgebaut werden.

Es ist auch bereits bekannt, Chemischreinigungsmaschinen in zeitlichen Abständen mit Hilfe von Formaldehyd zu dekontaminieren, was gewöhnlich nach Betriebsschluß erfolgt, um durch freiwerdende Formaldehyddämpfe möglichst wenig Personal zu gefährden. Diese Behandlung ist unbequem und für das Personal unangenehm. Außerdem müssen alle mit dem Formaldehyd in Berührung kommenden Maschinenteile anschließend gespült werden, und das Lösemittel muß destilliert werden, bevor es wieder zur weiteren Behandlung von Textilien verwendet wird, da andernfalls diese Textilien noch längere Zeit nach der Reinigung den stechenden Formaldehydgeruch aufweisen.

Die der Erfindung zugrundeliegende Aufgabe bestand somit darin, ein wirksames Mittel zur Verhinderung einer Kontamination von Chemischreinigungsmaschinen zu bekommen, das auch in der Gasphase der Maschine wirksam wird, bequem zu handhaben ist und einen unerwünschten Geruch der gereinigten Textilien vermeidet.

Das erfindungsgemäße Verfahren zur Desinfektion von Chemischreinigungsmaschinen, in denen ein Lösemittel zusammen mit einem Tensid als Reinigungsverstärker verwendet wird und ein desinfizierendes Mittel enthält, ist dadurch gekennzeichnet, daß man dem Lösemittel als desinfizierendes Mittel ein Harnstoffderivat der allgemeinen

Formel

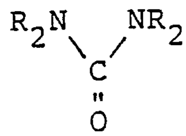

zusetzt, in der R ein Wasserstoffatom oder eine Hydroxymethylgruppe bedeutet, wobei wenigstens einer der Substituenten R eine Hydroxymethylgruppe ist.

Die erfindungsgemäß verwendeten Harnstoffderivate ergeben den überraschenden Vorteil, daß sie die Gasphase der Chemischreinigungsmaschine dekontaminieren und desinfizieren, indem sie an die als Reinigungsverstärker verwendeten Tenside gebunden werden und kolloidale Addukte bilden. Diese Addukte werden von den Textilfasern, besonders intensiv von Cellulosefasern, aber auch von Wolle, adsorbiert. Auf diese Weise gelangen die Harnstoffderivataddukte mit den Textilien in den Trocknungsbereich der Chemischreinigungsmaschine, wo sie durch die dortige Temperaturerhöhung sich zersetzen und langsam und kontinuierlich feine Mengen von Formaldehyd freisetzen, der die Entstehung einer mikrobiologischen Kontamination verhindert und gleichzeitig die Anreicherung im Kondenswasser bereits vorliegender mikrobiologischer Kontaminationen langsam (je nach den Anwendungsbedingungen innerhalb von zwei Tagen bis zu einer Woche) zerstört.

In dem Lösemittel verbleibendes kolloidal gelöstes Addukt von Reinigungsverstärker und Harnstoffderivat gelangt auch in den Destillationsbereich der Chemischreinigungsmaschine, wo innerhalb der Destillation ebenfalls Formaldehyd abgespalten wird, der in die Wasserphase des Wasserabscheiders gelangt.

Die Bindung des Harnstoffderivates an den Reinigungsverstärker hat den weiteren Vorteil, daß eine feine und genaue Dosierung des desinfizierenden Mittels möglich ist. So kann ein Gleichgewicht der Formaldehydkonzentration im Wasserabscheider knapp oberhalb der minimalen Hemmkonzentration von 0,05 bis 0,1 % dauernd gewährleistet werden.

Durch die feine Dosierung und die kontinuierliche langsame Formaldehydabgabe aus dem Harnstoffderivat läßt sich an den gereinigten Textilien im Anschluß an das Reinigungsverfahren kein Formaldehyd nachweisen (mit der Draeger-Gasspürpumpe), und es tritt kein Formaldehydgeruch auf, und zwar weder in den Räumen, wo die Chemischreinigungsmaschine aufgestellt ist, noch bei den gereinigten Textilien. Die Verwendung der erfindungsgemäßen desinfizierenden Mittel ist daher bequem und völlig ungefährlich für das Personal und erfordert nur äußerst geringen zusätzlichen Arbeitsaufwand. Die für das erfindungsgemäße Verfahren zu verwendenden Mittel fallen nicht unter die Gefahrstoffverordnung.

Als Harnstoffderivate können nach der Erfindung solche der obigen Formel mit 1 bis 4 Hydroxymethylgruppen verwendet werden. Damit können diese Harnstoffderivate einzeln oder im Gemisch miteinander eingesetzt werden. Bevorzugt verwendet man den N,N'-Di-(hydroxymethyl)-harnstoff.

Als Mittel zur Durchführung des erfindungsgemäßen Verfahrens kann das Harnstoffderivat als solches angesehen werden, das dem Lösemittel der erforderlichen Menge zudosiert wird. Das Mittel kann aber auch ein Trägermaterial, wie ein Lösemittel für das Harnstoffderivat und/oder das als Reinigungsverstärker verwendete Tensid enthalten.

Besonders günstig ist es, in dem erfindungsgemäßen Verfahren und Mittel als Reinigungsverstärker ein kationisches Tensid zu verwenden. Besonders bevorzugt sind dabei als kationische Tenside quaternäre Ammoniumsalze vom Typ der Difettalkyldimethylammoniumsalze, Fettalkylbenzyldimethylammoniumsalze oder Triammoniumsalze der Citronensäure oder Tricarbalylsäure. Diese Reinigungsverstärker sind in den DE-PSen 3 111 158 und 3 111 149 beschrieben. Diejenigen Reinigungsverstärker mit desinfizierender Wirkung, wie jene gemäß der DE-PS 3 111 158, vermindern die erforderliche Konzentration an Formaldehydzugabe weiter, was von besonderem Vorteil ist.

Während im vorliegenden Verfahren bei Verwendung anionischer Tenside als Reinigungsverstärker, die mit einer mittleren Konzentration von 5 ml je Liter Lösemittel eingesetzt werden, ein Gehalt von 0,5 bis 1 % des Harnstoffderivates erforderlich ist, genügt in Verbindung mit kationischen Tensiden gemäß der DE-PS 3 111 158 ein Zusatz von nur 0,05 bis 0,2 Gew.-% des Harnstoffderivates. Dies zeigt, daß die Kombination der erfindungsgemäß verwendeten Harnstoffderivate mit kationischen Tensiden, insbesondere jenen gemäß der DE-PS 3 111 158 einen besonders vorteilhaften, ja synergist schen Effekt ergibt. Die chemische Konstitution dieser im vorliegenden Verfahren besonders günstigen Reinigungsverstärker läßt sich gemäß Patentanspruch 1 der DE-PS 3 111 158 definieren. Auf diese Definition wird hier Bezug genommen.

Weiterhin ist es zweckmäßig, dem Lösemittel oder dem Mittel zur Durchführung des erfindungsgemäßen Verfahrens 1,6-Dihydroxy-2,5-dioxohexan und/oder wenigstens ein Isothiazolinonderivat zuzusetzen.

Die folgenden Beispiele dienen der weiteren Erläuterung der Erfindung.

Beispiel 1

Dem Reinigungsverstärker A aus Beispiel 1, Spalte 5 der DE-PS 3 111 158 wurden 0,2 % N,N'-Di-(hydroxymethyl)-harnstoff in der Weise zugesetzt, daß der Zusatz dem Wassergehalt des Reinigungsverstärkers zugefügt wurde, bevor dieses Wasser bei der Herstellung des Reinigungsverstärkers durch die Grenzflächenaktivität der übrigen Wirkstoffe im Reinigungsverstärker kolloidal gebunden wurde.

Der so modifizierte Reinigungsverstärker wurde in einer handelsüblichen Reinigungsmaschine in der allgemein üblichen Konzentration mit 5 ml je Liter Lösemittel zur Reinigung von Textilien eingesetzt. Nach jedem Reinigungsvorgang wurde der Formaldehydgehalt der gereinigten Textilien mit Draeger-Prüfröhrchen bestimmt. Parallel dazu wurde die Formaldehydkonzentration in der Wasserphase des Wasserabscheiders gemessen.

Es wurden 100 Chargen à 10 kg Reinigungsgut (etwa zur Hälfte die in Reinigungsbetrieben übliche Warenmischung, zur Hälfte Polizeiuniformen) gereinigt. Bei keiner der Chargen wurde unmittelbar nach dem Entladen der Maschine im Draeger-Prüfröhrchen Formaldehyd angezeigt. Das Personal, das die Kleidung anschließend zu bügeln hatte, wurde gebeten, besonders auf stechenden Geruch oder Augentränen zu achten. Es wurden keinerlei Beobachtungen diesbezüglich gemacht.

Der Formaldehydgehalt im Wasserabscheider betrug nach 5 Chargen 0,03 %, nach 10 Chargen 0,11 %, nach 20 Chargen 0,19 %, nach 50 Chargen 0,24 %, nach 100 Chargen 0,28 %.

Beispiel 2

In einer massiv verkeimten Reinigungsmaschine, die mit Trichlortrifluorethan (R 113) betrieben wurde, wurde der in Beispiel 1 beschriebene erfindungsgemäße Reinigungsverstärker mit 5 ml/l eingesetzt. Die massive Verkeimung dieser Maschine wirkte sich insbesondere dahingehend aus, daß an gereinigten Textilien ein intensiver muffiger Geruch verursacht wurde. Um die Wirkung des erfindungsgemäßen Verfahrens zu überwachen, wurden Testgewebe aus Baumwollcord von 40 x 40 cm Größe jeder Charge zugefügt, und die Geruchsbeeinträchtigung wurde anschließend von mehreren Personen bestimmt.

Innerhalb der ersten 3 Chargen nach Einsatz des erfindungsgemäßen Reinigungsverstärkers wurde keine Veränderung festgestellt. Die Testgewebe wurden ingleichem Maße als unangenehm riechend beurteilt.

Nach der fünften Charge glaubten einige der die Prüfung vornehmenden Personen, daß eine leichte Minderung des unangenehmen Geruches eingetreten sei.

Nach der zehnten Charge beurteilten alle Prüfpersonen, daß eine deutliche Geruchsverminderung eingetreten war.

Nach der zwanzigsten Charge wurde kein Fremdgeruch mehr festgestellt.

Beispiel 3 und Vergleichsbeispiel 1

Einer Reinigungsmaschine, die mit Tetrachlorethan betrieben wurde, wurde zur Aufbereitung des Abwasser ein "Sicherheitsabscheider" nachgeschaltet, der in der Trennkammer ein Volumen von120 1 Wasser besaß. Die Reinigungsmaschine wurde einen Monat lang mit dem Reinigungsverstärker A entsprechend der DE-PS 3 111 158, jedoch ohne Zusatz des Harnstoffderivates, betrieben. Bereits nach etwa zweiwöchiger Nutzungsdauer wurde das Wasser im Sicherheitsabscheider trübe und nahm einen zunehmend muffiger werdenden Geruch an. Die in dieser Maschine gereinigten Textilien - vor allem solche aus Baumwolle - wurden häufig beanstandet, weil sie "muffig" rochen.

Im Anschluß an diesen vierwöchigen Einsatz wurde ohne Veränderung der Maschine und des Reinigungsverfahrens der Reinigungsverstärker durch das in Beispiel 1 beschriebene erfindungsgemäße Mittel ersetzt.

Bereits nach dreitägigem Einsatz war die Wasserphase im Sicherheitsabscheider wieder klar, Fremdgeruch war nicht mehr festzustellen. Das in der Maschine gereinigte Reinigungsgut roch neutral - ohne Fremdgeruch. Der Formaldehydgehalt in der Wasserphase des Sicherheitsabscheiders betrug 0,06 %.

Vergleichsbeispiel 2

Zu Vergleichszwecken wurde ein Reinigungsverstärker eingesetzt, dem 1,5 % Formaldehyd zugesetzt war. Vorgesehen war eine Versuchsdauer über 100 Chargen. Bereits nach 5 Chargen mußte der Versuch abgebrochen werden, weil das Personal über Schleimhautreizung und Augentränen klagte, wenn die Maschine entladen wurde. Der Formaldehydgehalt im Wasserabscheider der Reinigungsmaschine betrug zu diesem Zeitpunkt dennoch nur 0,05 %.

Vergleichsbeispiel 3

Einem handelsüblichen anionischen Reinigungsverstärker gemäß Beispiel 4 der DE-PS 2 412175, Substanz L, wurden 0,2 % N,N′-Di-(hydroxymethyl)-harnstoff in gleicher Weise, wie in Beispiel 1 beschrieben, zugesetzt. Beim Einsatz dieses Reinigungsverstärkers in eine mikrobiologisch kontaminierte Reinigungsmaschine wurde während 20 Chargen keine Verbesserung des Betriebszustandes festgestellt. Daraufhin wurde die Konzentration des Harnstoffderivates auf 1,0 % erhöht. Erst nach dieser Konzentrationserhöhung trat innerhalb der nächsten 20 Chargen Zug um Zug eine Beseitigung des muffigen Geruches ein. Die Formaldehydkonzentration in der Wasserphase des Wasserabscheiders lag nach den 20 Chargen bei 0,31 %.

Formaldehydgeruch am Reinigungsgut wurde nicht bemerkt. Mit Draeger-Prüfröhrchen war Formaldehyd nicht feststellbar.

## Ansprüche

1. Verfahren zur Desinfektion von Chemischreinigungsmaschinen, in denen ein Lösemittel zusammen mit einem Tensid als Reinigungsverstärker verwendet wird und ein desinifizierendes Mittel enthält, **dadurch gekennzeichnet,** daß man dem Lösemittel als desinfizierendes Mittel ein Harnstoffderivat der allgemeinen Formel

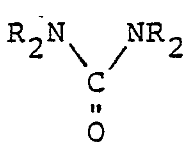

zusetzt, in der R ein Wasserstoffatom oder eine Hydroxymethylgruppe bedeutet, wobei wenigstens einer der Substituenten R eine Hydroxymethylgruppe ist.

2. Verfahren nach Anspruch 1, **dadurch gekennzeichnet,** daß man als Harnstoffderivat N,N′-Di-(hydroxymethyl)-harnstoff verwendet.

3. Verfahren nach Anspruch 1 oder 2, **dadurch gekennzeichnet,** daß man als Reinigungsverstärker ein kationisches Tensid verwendet.

4. Verfahren nach Anspruch 3, **dadurch gekennzeichnet,** daß man als kationisches Tensid wenigstens ein quaternäres Ammoniumsalz vom Typ der Difettalkyldimethylammoniumsalze, Fettalkylbenzyldiemthylammoniumsalze oder Triammoniumsalze der Citronensäure oder Tricarbalylsäure verwendet.

5. Verfahren nach einem der Ansprüche 3 und 4, **dadurch gekennzeichnet,** daß man das Harnstoffderivat in einer Menge von 0.01 bis 0,5, vorzugsweise von 0,03 bis 0,3 besonders von 0.05 bis 0,2 % (Gewicht/Volumen), bezogen auf das Volumen des verwendeten Lösemittels, zusetzt.

6. Verfahren nach einem der Ansprüche 1 bis 5, **dadurch gekennzeichnet,** daß man dem Lösemittel zusätzlich ein Isothiazolinonderivat zusetzt.

7. Verfahren nach einem Ansprüche 1 bis 6, **dadurch gekennzeichnet,** daß man dem Lösemittel zusätlich 1,6-Dihydroxy-2,5-dioxohexan zusetzt.

8. Mittel zur Durchführung des Verfahrens nach einem der Ansprüche 1 bis 7 mit einem desifizierenden Mittel, **dadurch gekennzeichnet,** daß es als desinfizierendes Mittel ein Harnstoffderivat der allgemeinen Formel

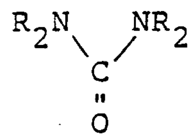

zusetzt, in der R ein Wasserstoffatom oder eine Hydroxymethylgruppe bedeutet, wobei wenigstens einer der Substituenten R eine Hydroxymethylgruppe ist.

9. Mittel nach Anspruch 8, **dadurch gekennzeichnet,** daß es zusätzlich ein Lösemittel für das Harnstoffderivat enthält.

10. Mittel nach Anpruch 8 oder 9, **dadurch gekennzeichnet,** daß es zusätzlich ein Tensid, vorzugsweise ein kationisches Tensidm enthält.

11. Mittel nach Anspruch 10, **dadurch gekennzeichnet,** daß es als kationisches Tensid wenigstens ein quaternäres Ammoniumsalz vom Typ der Defettalkyldimethylammoniumsalze, Fettalkylbenzyldimethylammoniumsalze oder Triammoniumsalze der Citronensäure oder Tricarbalylsäure enthält.

12. Mittel nach einem der Ansprüche 8 bis 11, **dadurch gekennzeichnet,** daß es zusätzlich ein Isothiazolinonderi vat enthält.

13. Mittel nach einem der Ansprüche 8 bis 12, **dadurch gekennzeichnet,** daß es zusätzlich 1,6-Dihydroxy-2,5-dioxohexan enthält.